(19) **Europäisches Patentamt**
**European Patent Office**
**Office européen des brevets**

(11) **EP 3 183 363 B1**

(12) **FASCICULE DE BREVET EUROPEEN**

(45) Date de publication et mention
de la délivrance du brevet:
**18.03.2020 Bulletin 2020/12**

(51) Int Cl.:
*G01N 33/574* (2006.01)          *G01N 33/68* (2006.01)
*C12Q 1/68* (2018.01)          *C12Q 1/6886* (2018.01)

(21) Numéro de dépôt: **15759902.8**

(22) Date de dépôt: **13.08.2015**

(86) Numéro de dépôt international:
**PCT/FR2015/052207**

(87) Numéro de publication internationale:
**WO 2016/027029 (25.02.2016 Gazette 2016/08)**

(54) **PROCÉDÉ POUR DÉTERMINER LE PRONOSTIC DE SURVIE D'UN PATIENT ATTEINT D'UN CANCER DU PANCRÉAS**

VERFAHREN ZUR BESTIMMUNG DER ÜBERLEBENSPROGNOSE EINES PATIENTEN MIT PANKREASKREBSLEIDEN

METHOD FOR DETERMINING THE SURVIVAL PROGNOSIS OF A PATIENT SUFFERING FROM PANCREATIC CANCER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.08.2014 FR 1457934**

(43) Date de publication de la demande:
**28.06.2017 Bulletin 2017/26**

(73) Titulaire: **ACOBIOM**
**34790 Grabels (FR)**

(72) Inventeur: **PIQUEMAL, David**
**F-30380 Saint Christol Les Ales (FR)**

(74) Mandataire: **Santarelli**
**49, avenue des Champs-Elysées**
**75008 Paris (FR)**

(56) Documents cités:
• A. TSIAOUSIDOU ET AL: "B7H4, HSP27 and DJ-1 molecular markers as prognostic factors in pancreatic cancer", PANCREATOLOGY, vol. 13, no. 6, 1 novembre 2013 (2013-11-01), pages 564-569, XP055202137, ISSN: 1424-3903, DOI: 10.1016/j.pan.2013.10.005

• Hayato Fujita ET AL: "Gene expression levels as predictive markers of outcome in pancreatic cancer after gemcitabine-based adjuvant chemotherapy", Neoplasia (New York, N.Y.), 1 octobre 2010 (2010-10-01), page 807, XP055158050, Canada DOI: 10.1593/neo.10458 Extrait de l'Internet: URL:http://www.ncbi.nlm.nih.gov/pubmed/209 27319

• KRACMAROVA ALZBETA ET AL: "High expression of ERCC1, FLT1, NME4 and PCNA associated with poor prognosis and advanced stages in myelodysplastic syndrome", LEUKEMIA AND LYMPHOMA, INFORMA HEALTHCARE, US, vol. 49, no. 7, 1 juillet 2008 (2008-07-01), pages 1297-1305, XP009133789, ISSN: 1042-8194

• AFFYMETRIX: "GeneChip TM Human Genome U133 Arraysge/pjp", DATASHEET AFFYMETRIX,, 1 janvier 2003 (2003-01-01), pages 1-8, XP007921348,

• TATSUYUKI TAKADATE ET AL: "Nm23/nucleoside diphosphate kinase-A as a potent prognostic marker in invasive pancreatic ductal carcinoma identified by proteomic analysis of laser micro-dissected formalin-fixed paraffin-embedded tissue", CLINICAL PROTEOMICS, BIOMED CENTRAL LTD, LONDON, UK, vol. 9, no. 1, 27 juin 2012 (2012-06-27), page 8, XP021139720, ISSN: 1559-0275, DOI: 10.1186/1559-0275-9-8

**Description**

La présente invention se rapporte au cancer du pancréas, et plus particulièrement au pronostic de survie d'un patient atteint d'un cancer du pancréas.

L'invention vise aussi à déterminer la capacité dudit patient à recevoir un traitement du cancer du pancréas, particulièrement un traitement à la gemcitabine.

Avec 43920 nouveaux cas annuels aux États-Unis, et 37390 décès, la mortalité liée au cancer du pancréas est de plus de 85%, ce qui place le cancer du pancréas au quatrième rang en termes de mortalités des cancers aux États-Unis tant chez les hommes que chez les femmes.

L'incidence du cancer du pancréas a nettement augmenté au cours des dernières décennies. Chaque année, environ 60000 personnes en Europe, et plus de 230000 dans le monde entier, sont diagnostiqués avec cette maladie.

Les patients atteints d'un cancer du pancréas présentent souvent un moins bon pronostic par rapport à d'autres tumeurs malignes et types de cancer, en partie parce que la détection précoce est difficile.

Au moment du diagnostic, la plupart des patients atteints d'un adénocarcinome canalaire pancréatique présente une maladie localement avancé ou métastatique, et seulement 10 à 20% des cas sont des candidats à une chirurgie curative.

La médiane de survie à partir du diagnostic est d'environ 3 à 6 mois; la survie à 5 ans est très inférieure à 5% et une rémission complète est extrêmement rare.

Les thérapies actuelles approuvées ou utilisées en pratique clinique chez les patients atteints de cancer du pancréas sont des traitements à base de gemcitabine, folfirinox ou encore d'erlotinib.

La gemcitabine est un analogue de nucléoside, administré par voie intraveineuse à une dose d'environ 1000 mg/m$^2$. Un traitement à la gemcitabine fait usuellement partie des traitements de première intention recommandés pour le cancer du pancréas résécable (phase I-II), le cancer du pancréas localement avancé non résécable (stade III) et du cancer du pancréas métastatique avancé (stade IV).

Avec la gemcitabine, la médiane de survie globale varie entre 4,9 mois et 8,3 mois.

Le succès limité des traitements et le taux de mortalité qui reste élevé pour une partie des patients soulignent la nécessité en moyens pour améliorer le ciblage thérapeutique des patients qui pourraient s'avérer de bons répondeurs à la gemcitabine et qui toléreraient bien la gemcitabine dans cette indication.

Il est décrit dans l'art antérieur une corrélation entre la survie d'un patient atteint d'un cancer du pancréas et l'expression dans ses cellules tumorales de certains marqueurs (A. Tsiaousidou et al., Pancreatology, 2013, 13, pp. 564-569, H. Fujita et al., Neoplasia, 2010, 12, pp. 807-817 et T. Takadate et al., Clinical Proteomics, 2012, 9:8).

Le traitement du cancer du pancréas par différents composés peut avoir différents degrés d'efficacité en fonction du patient. Cependant, jusqu'à aujourd'hui, il n'existe aucun moyen de prédire facilement le bénéfice clinique des différents traitements disponibles sur un patient donné.

Il existe ainsi toujours un besoin en tests permettant de sélectionner pour chaque patient le bon traitement, de manière à leur donner les meilleures chances de survie.

Dans les cas de patients atteints de cancer du sang, il est possible d'établir un pronostic de survie de manière non invasive grâce à la mesure d'expression de marqueurs spécifiques dans un échantillon de sang périphérique, où sont localisées les cellules cancéreuses (A. Kracmarova et al., Leukemia & Lymphoma, 2008, 49 (7), pp. 1297-1305). Toutefois, dans le cas d'un cancer du pancréas, les cellules cancéreuses sont difficilement accessibles.

Il pourrait ainsi être souhaitable que ledit test soit un test que l'on puisse réaliser régulièrement. Un avantage supplémentaire serait que ledit test soit non invasif.

Le travail des inventeurs a permis d'identifier un ensemble de gènes dont l'étude du niveau d'expression permet d'évaluer le temps de survie d'un patient atteint d'un cancer du pancréas.

Avantageusement cette prédiction permet également de prédire les chances de réussite d'un traitement à la gemcitabine et de sélectionner les patients susceptibles de répondre favorablement à un traitement à la gemcitabine.

Ainsi l'étude du niveau d'expression desdits gènes permet d'apporter un enseignement quant à l'opportunité d'administrer un traitement à la gemcitabine à un patient atteint d'un cancer du pancréas.

Un des avantages de l'invention réside dans le fait que le niveau d'expression desdits gènes peut être évalué directement à partir d'un échantillon de sang, avantageusement de sang total du patient, préalablement prélevé.

Ceci est particulièrement avantageux dans le cas du cancer du pancréas car il est très difficile d'accéder aux cellules tumorales.

En outre, l'échantillonnage du sang (collecte, stabilisation et transport) peut être standardisé.

Enfin l'utilisation de sang total est plus sûre que l'utilisation d'une fraction de sang comme par exemple des cellules mononucléées du sang périphérique (PBMC), car elle permet d'éviter les erreurs de manipulation liées à la préparation desdites fractions. Par "sang total", on entend le sang avec tous ses constituants, (plasma, globules rouges, globules blancs, plaquettes, ...) par opposition à "fraction de sang", qui correspond à un produit obtenu par séparation des composants particuliers du sang. Les cellules mononucléaires du sang périphérique constituent un exemple d'une fraction de sang.

L'invention a donc pour objet un procédé *in vitro* pour établir un pronostic de survie chez un patient atteint d'un cancer du pancréas comprenant les étapes suivantes :

a) mesurer le niveau d'expression de chaque gène d'une combinaison de gènes marqueurs choisis parmi le groupe constitué des gènes *NME4* ou un gène homologue de celui-ci et *ARL4C* ou un gène homologue de celui-ci, lesdits gènes homologues étant des gènes présentant un degré d'identité de séquence d'au moins 90% avec le gène *NME4* ou *ARL4C* respectivement, dans un échantillon de sang dudit patient préalablement prélevé ;
b) comparer le niveau d'expression mesuré des gènes marqueurs choisis à l'étape a) chez ledit patient avec un seuil de référence ;
c) évaluer le temps de survie chez ledit patient.

Le terme «homologue» est défini dans le présent texte comme une séquence de polynucléotides présentant un degré d'identité d'au moins 90%, et préférentiellement de 99% avec la séquence du gène (de longueur totale) sauvage. Le degré d'identité fait référence à une identité de séquence entre deux séquences. L'identité peut être déterminée en comparant une position dans chaque séquence qui peut être alignée à des fins de comparaison. Quand une position équivalente dans les séquences comparées est occupée par la même base, alors les molécules sont identiques à cette position. Divers algorithmes et / ou programmes d'alignement peuvent être utilisés pour la détermination de l'homologie de deux séquences, dont FASTA et BLAST.

Le procédé selon l'invention permet d'établir un pronostic de survie chez un patient atteint d'un cancer du pancréas.

Selon l'invention, les étapes a) à c) du procédé ci-avant peuvent être mises en œuvre sur un patient atteint d'un cancer du pancréas ayant reçu un traitement, et particulièrement un traitement à base de gemcitabine, pour prévoir ou évaluer l'efficacité et/ou le bénéfice d'un traitement du cancer du pancréas chez un patient atteint de ladite pathologie, en particulier, en termes d'espérance de vie.

Ainsi l'invention a aussi pour objet un procédé *in vitro* pour prévoir ou évaluer l'efficacité et/ou le bénéfice d'un traitement du cancer du pancréas chez un patient atteint de ladite pathologie comprenant les étapes suivantes :

a) mesurer le niveau d'expression de chaque gène d'une combinaison de gènes marqueurs choisis parmi le groupe constitué des gènes *NME4* ou un gène homologue de celui-ci et *ARL4C* ou un gène homologue de celui-ci, lesdits gènes homologues étant des gènes présentant un degré d'identité de séquence d'au moins 90% avec le gène *NME4* ou *ARL4C* respectivement, dans un échantillon de sang dudit patient préalablement prélevé ;
b) comparer le niveau d'expression mesuré des gènes marqueurs choisis à l'étape a) chez ledit patient avec un seuil de référence;
c) évaluer le temps de survie chez ledit patient.

Le procédé selon l'invention est particulièrement adapté pour prévoir ou évaluer l'efficacité et/ou le bénéfice d'un traitement du cancer du pancréas à la gemcitabine.

Dans les procédés selon l'invention, le seuil de référence utilisé à l'étape c) pour effectuer la comparaison est préalablement déterminé pour chaque gène ou chaque combinaison de gènes.

Le principe de l'invention repose sur le fait que la Demanderesse a pu établir que l'étude du niveau d'expression d'au moins l'un des 5 gènes (*NME4, ITPR3, SESN3, ARL4C* et *RPLP1*), avantageusement d'une combinaison de 2 à 5 de ces gènes, encore plus avantageusement d'une combinaison des 5 gènes, permet d'établir un pronostic de survie du patient. Plus particulièrement, La Demanderesse a établi qu'en comparant des données de niveau d'expression d'au moins l'un des 5 gènes (*NME4, ITPR3, SESN3, ARL4C* et *RPLP1*) ou leur combinaison, obtenues à partir d'un échantillon de sang dudit patient testé avec des données qu'elle a elle-même établies pour les 5 gènes considérés ou leur combinaison sur une population référence de malades atteints d'un cancer du pancréas, avant ou pendant le traitement, permettait d'établir un pronostic de survie du patient et un suivi dans le traitement de celui-ci.

Le procédé selon l'invention peut être réalisé sur un échantillon de sang d'un patient, de préférence sur un échantillon de sang périphérique dudit patient, ledit échantillon de sang étant prélevé préalablement à la mise en œuvre du procédé selon l'invention. Le sang périphérique est le sang qui circule à travers le cœur, les artères, les capillaires et les veines.

Selon la divulgation, l'étape a) du procédé peut être réalisée en mesurant le niveau d'expression de 1 des 5 gènes (ou gènes homologues) du groupe défini ci-avant, avantageusement de 2 des 5 gènes, plus avantageusement de 3 des 5 gènes, encore plus avantageusement de 4 des 5 gènes, et préférentiellement des 5 gènes (ou gènes homologues) du groupe défini ci-avant.

Selon l'invention, l'étape a) du procédé peut être réalisée en mesurant le niveau d'expression des gènes *NME4* et *ARL4C* (ou gènes homologues), plus avantageusement de 3 des 5 gènes (ou gènes homologues) du groupe défini ci-avant dont *NME4* et *ARL4C*, encore plus avantageusement de 4 des 5 gènes (ou gènes homologues) du groupe défini ci-avant dont *NME4* et *ARL4C*, et préférentiellement des 5 gènes (ou gènes homologues) du groupe défini ci-avant.

Selon la divulgation l'étape a) du procédé peut être réalisée en mesurant le niveau d'expression d'au moins un gène (ou gène homologue) choisi parmi les gènes *NME4*, *ITPR3*, *SESN3*, *ARL4C* et *RPLP1*, avantageusement d'au moins une des combinaisons suivantes : *NME4* et *ITPR3* ; *NME4* et *SESN3* ; *NME4* et *ARL4C* ; *NME4* et *RPLP1* ; *ITPR3* et *SESN3* ; *ITPR3* et *ARL4C* ; *ITPR3* et *RPLP1* ; *SESN3* et *ARL4C* ; *SESN3* et *RPLP1* ; *ARL4C* et *RPLP1* ; plus avantageusement le niveau d'expression des gènes de l'une des combinaisons suivantes : *NME4* et *ITPR3* et *SESN3* ; *NME4* et *ITPR3* et *ARL4C* ; *NME4* et *ITPR3* et *RPLP1* ; *NME4* et *SESN3* et *ARL4C* ; *NME4* et *SESN3* et *RPLP1* ; *NME4* et *ARL4C* et *RPLP1* ; *ITPR3* et *ARL4C* et *RPLP1* ; *ITPR3* et *SESN3* et *RPLP1* ; *ITPR3* et *SESN3* et *ARL4C* ; *SESN3* et *ARL4C* et *RPLP1* ; encore plus avantageusement le niveau d'expression des gènes de l'une des combinaisons suivantes *NME4* et *ITPR3* et *SESN3* et *ARL4C* ; *NME4* et *ITPR3* et *ARL4C* et *RPLP1* ; *NME4* et *ITPR3* et *SESN3* et *RPLP1* ; *ITPR3* et *SESN3* et *ARL4C* et *RPLP1*, et préférentiellement la combinaison suivante *NME4* et *ITPR3* et *SESN3* et *ARL4C* et *RPLP1*.

Ainsi selon l'invention l'étape a) du procédé peut être réalisée en mesurant le niveau d'expression avantageusement d'au moins la combinaison suivante : *NME4* et *ARL4C* ; plus avantageusement le niveau d'expression des gènes de l'une des combinaisons suivantes : *NME4* et *ITPR3* et *ARL4C* ; *NME4* et *SESN3* et *ARL4C* ; *NME4* et *ARL4C* et *RPLP1* ; encore plus avantageusement le niveau d'expression des gènes de l'une des combinaisons suivantes *NME4* et *ITPR3* et *SESN3* et *ARL4C* ; *NME4* et *ITPR3* et *ARL4C* et *RPLP1,* et préférentiellement la combinaison suivante *NME4* et *ITPR3* et *SESN3* et *ARL4C* et *RPLP1.*

Préférentiellement, selon l'invention, l'étape a) du procédé est réalisée en mesurant le niveau d'expression des deux gènes (ou gène homologue) *NME4 et ARL4C* ou le niveau d'expression des gènes de la combinaison suivante *NME4* et *ITPR3* et *SESN3* et *ARL4C* et *RPLP1.*

Dans un premier mode de réalisation du procédé selon l'invention, le niveau d'expression des gènes *NME4* et *ARL4C*, et optionnellement du ou des gène(s) choisi dans le groupe consistant en *ITPR3*, *SESN3* et *RPLP1* est mesuré par la mesure de la quantité de la protéine codée par ledit gène dans l'échantillon de sang dudit patient à tester. Dans ce cas, le taux de protéines est de préférence mesuré en utilisant des méthodes de détection à base d'anticorps tels que l'immunochimie ou l'analyse western-blot.

Dans un autre mode de réalisation du procédé selon l'invention, le niveau d'expression des gènes est mesuré par la mesure du taux de transcription dudit gène. Ce taux peut être mesuré par la mesure de la quantité de l'ARN ou de l'ADNc dudit gène. Dans ce cas, le niveau d'ARN transcrit ou l'ADNc peut être mesuré en utilisant des méthodes de détection d'acides nucléiques comme la PCR quantitative, les puces à ADN, l'hybridation avec des sondes marquées, ou les immuno-essais en écoulement latéral, en particulier à l'aide de bandelettes d'écoulement latéral.

De préférence, dans les procédés selon l'invention, le niveau d'expression du gène ou de la combinaison de gènes, est mesuré par PCR quantitative en temps réel (*real time quantitative polymerase chain réaction* ou qPCR) réalisée sur les transcrits ARN dudit gène ou sur leur ADN complémentaire (cDNA). Une qPCR est une PCR dans laquelle l'ADN amplifié est détecté en temps réel alors que la réaction de polymérisation progresse. Cette détection se fait par l'accumulation d'un signal fluorescent.

Cette technique utilise des amorces (sens et anti-sens) et un marqueur, de préférence, un intercalant fluorescent de l'ADN formé au cours des cycles de PCR. Avantageusement, lesdites amorces sont spécifiques des régions flanquantes du gène considéré.

Dans le cas du gène *NME4,* les régions flanquantes sont situées sur le chromosome 16 entre les nucléotides 399665 et 399728 (Assembly Dec. 2013, GRch38/hg38 source UCSC).

Dans le cas du gène *ITPR3*, les régions flanquantes sont situées sur le chromosome 6 entre les nucléotides 33696404 et 33696485 (Assembly Dec. 2013, GRch38/hg38 décembre 2013, source UCSC).

Dans le cas du gène *SESN3,* les régions flanquantes sont situées sur le chromosome 11 entre les nucléotides 95166241 et 95166313 (Assembly Dec. 2013, GRch38/hg38, source UCSC).

Dans le cas du gène *ARL4C,* les régions flanquantes sont situées sur le chromosome 4 entre les nucléotides 234 493 401 et 234 493 502 (Assembly Dec. 2013, GRch38/hg38 décembre 2013, source UCSC).

Dans le cas du gène de la *RPLP1,* les régions flanquantes sont situées sur le chromosome 15 entre les nucléotides 69455485 et 69455598 (Assembly Dec. 2013, GRch38/hg38 décembre 2013, source UCSC).

Dans un mode de réalisation avantageux, les amorces suivantes peuvent être utilisées pour réaliser la qPCR :

- ARNs issus du gène *NME4*

  ∘ amorce sens: CATGATTGGACACACCGACTC (SEQ ID NO. 1)
  ∘ amorce anti-sens: GACGCTGAAGTCACCCCTTAT (SEQ ID NO. 2)

- ARNs issus du gène *ITPR3*

○ amorce sens: TCCTGGGGAAGAGTTGTACG (SEQ ID NO. 3)
○ amorce anti-sens: AGGAGAAAAACAAGCGGTCA (SEQ ID NO. 4)

- ARNs issus du gène *SESN3*

○ amorce sens: TTGCCTTTGTAGTCCTGTGC (SEQ ID NO. 5)
○ amorce anti-sens: CATTAGTCCAGTCACGTGCTTC (SEQ ID NO. 6)

- ARNs issus du gène ARLC4

○ amorce sens: AAAGCCCTGTGGTGTATCAA (SEQ ID NO. 7)
○ amorce anti-sens: GCTTCCTCTGTTGGGTCAGA (SEQ ID NO. 8)

- ARNs issus du gène *RPLP1*

○ amorce sens: TGGGCTTTGGTCTTTTTGAC (SEQ ID NO. 9)
○ amorce anti-sens: CAGACCATTTTTGCAGAGCA (SEQ ID NO. 10)

La qPCR permet de déterminer la valeur du nombre de cycle (Ct) d'un gène chez le patient à tester. Le Ct («cycle threshold») est défini comme le nombre de cycles de PCR nécessaires pour que le signal fluorescent franchisse le bruit de fond.

La valeur de Ct obtenue peut être ensuite normalisée par rapport au niveau d'expression d'au moins un gène ubiquitaire exprimé en nombre de cycle dans le même échantillon de sang du même patient. Lesdits gènes ubiquitaires sont des gènes qui sont exprimés dans toutes les cellules d'un organisme dans des conditions normales et physiopathologiques. Ces gènes sont généralement exprimés à des niveaux relativement constants.

De préférence, dans les procédés selon l'invention, à l'étape a), outre le niveau d'expression de chaque gène (Ct_{gène}) d'une combinaison d'au moins 2 gènes marqueurs, on mesure le niveau d'expression d'au moins un gène ubiquitaire, lesdits niveaux d'expression étant mesurés en nombre de cycle(s) (Ct, « cycle threshold ») par PCR quantitative en temps réel (qPCR), puis on normalise le niveau d'expression des gènes marqueurs par rapport au niveau d'expression du au moins un gène ubiquitaire selon

$$\Delta Ct_{gène} = Ct_{gène} - [(\text{moyenne des}) \, Ct \, (\text{gène(s) ubiquitaire(s)})]$$

afin d'obtenir un niveau d'expression normalisé $\Delta Ct_{gène}$ pour chaque gène marqueur dont on a mesuré le niveau d'expression.

De préférence, dans les procédés selon l'invention, la normalisation est effectuée par rapport au niveau d'expression de deux gènes ubiquitaires, en particulier, par rapport au niveau d'expression des gènes GAPDH et B2M. Dans ce cas, le niveau d'expression normalisé $\Delta Ct_{gène}$ d'un gène marqueur est calculé par :

$$\Delta Ct_{gène} = Ct_{gène} - (Ct_{B2M} + Ct_{GAPDH}) / 2$$

Dans le cas du gène B2M, les régions flanquantes sont situées sur le chromosome 15 entre les nucléotides 44718721 et 44718792 (Assembly Dec. 2013, GRch38/hg38, source UCSC).

Dans le cas du gène de la GAPDH, les régions flanquantes sont situées sur sur le chromosome 12 entre les nucléotides et les nucléotides 6534833 6536572 (Assembly Dec. 2013, GRch38/hg38, source UCSC).

Des amorces particulièrement appropriées pour les gènes de *B2M* et *GAPDH* peuvent être:

- ARNs issus du gène *B2M*

○ amorce sens: GCTCAGTAAAGACACAACCATCC (SEQ ID NO. 11)
○ amorce anti-sens: CATCTGTGGATTCAGCAAACC (SEQ ID NO. 12)

- ARNs issus du gène *GAPDH*

○ amorce sens: ATGGGGAAGGTGAAGGTCG (SEQ ID NO. 13)
○ amorce anti-sens: GGGGTCATTGATGGCAACAATA (SEQ ID NO. 14)

Avantageusement, pour réaliser la qPCR, les amorces, leur taille (de préférence compris entre 80 et 150 nucléotides), leur température de fusion (Tm, de préférence de 60°C ± 1°C), leur pourcentage en nucléotides G et C (GC%, de préférence -60% en 3'), leur auto-complémentarité 3' et 5' et leur stabilité (de préférence inférieure à 4 nucléotides), les gammes de dimensions du produit et les paramètres thermodynamiques (évolution de la structure secondaire selon le Tm des amorces et la concentration en sel de sodium) sont choisis pour permettre une détection simultanée.

Selon un premier mode de réalisation de la divulgation, lors de l'étape a), le niveau d'expression d'un seul gène marqueur est mesuré. Dans ce mode de réalisation, lors des étapes b) et c), les seuils de référence (Sref) d'un gène donné sont définis dans le tableau 1 ci-après :

Tableau 1

| Gène | $S_{ref}$ |
|---|---|
| NME4 | 3,1864 |
| ITPR3 | 6,4014 |
| SESN3 | 3,6262 |
| ARL4C | 2,6286 |
| RPLP1 | 6,3748 |

- un niveau d'expression normalisé ΔCt de NME4 supérieur à 3,1864, et/ou
- un niveau d'expression normalisé ΔCt de ITPR3, SESN3, ARM4C et/ou
- RPLP1 inférieur respectivement à 6,4015 ; 3,6262 ; 2,6286 et 6,3748, indiquant un pronostic de survie à long terme, tandis que
- un niveau d'expression normalisé ΔCt de NME4 inférieur à 3,1864, et/ou
- un niveau d'expression normalisé ΔCt de ITPR3, SESN3, ARM4C et/ou
- RPLP1 supérieur respectivement à 6,4015 ; 3,6262 ; 2,6286 et 6,3748, indiquant un pronostic de survie à court terme

Par "survie à long terme", il est entendu la survie de plus de 10 mois, de préférence plus de 12 mois, encore plus préférablement plus de 15 mois.

Par "survie à court terme", on entend une survie de moins de 6 mois, moins de 5 mois, soit moins de 3 mois.

De plus, lorsqu'un patient est supposé avoir une survie à long terme, cela signifie également qu'il peut être considéré éligible pour un traitement à base de gemcitabine. A contrario, une survie à court terme du patient indique qu'un traitement à base de gemcitabine ne présentera pas ou peu de bénéfice au patient. Un autre traitement devrait alors être envisagé.

Les travaux de la Demanderesse (voir exemple 1) sur une population de référence l'ont conduite en effet à identifier 5 gènes pouvant être utilisés comme marqueurs pour un pronostic de survie d'un patient atteint d'un cancer du pancréas, mais également à déterminer pour ces 5 gènes, des valeurs pour des seuils de Seuil référence ($S_{ref}$) permettant d'effectuer une comparaison.

Toutefois, une plus grande précision dans le pronostic peut être apportée grâce à la mesure d'une combinaison d'au moins deux gènes. Par conséquent, selon un second mode de réalisation selon l'invention, lors de l'étape a), on mesure de niveau d'expression d'une combinaison d'au moins deux gènes marqueurs et, lors des étapes b), pour chaque gène marqueur dont on a mesuré l'expression :

i) on détermine une valeur $INDEX_{gène}$ dépendante du niveau d'expression normalisé du gène, définie par :

- $INDEX_{gène}$ = +1 *coefficient β du gène, si $\Delta Ct_{gène}$ > seuil de référence du gène, et
- $INDEX_{gène}$ = -1*coefficient β du gène, si $\Delta Ct_{gène}$ < seuil de référence du gène,

dans lequel le coefficient β et le seuil de référence d'un gène donné sont tels que définis dans le tableau 2 ci-après ;

Tableau 2

| Gène | $S_{ref}$ | Coefficient β |
|---|---|---|
| NME4 | 3,1864 | -1,3207 |
| ITPR3 | 6,4014 | 1,0850 |
| SESN3 | 3,6262 | 1,0301 |

(suite)

| Gène | $S_{ref}$ | Coefficient $\beta$ |
|---|---|---|
| *ARL4C* | 2,6286 | 1,3577 |
| *RPLP1* | 6,3748 | 1,0101 |

ii) on détermine, pour un patient donné, une valeur $INDEX_{patient}$ égale à la somme des $INDEX_{gène}$ des gènes de la combinaison, et

iii) on compare l'$INDEX_{patient}$ avec un seuil final de référence préalablement déterminé pour la combinaison de gènes.

La Demanderesse a ainsi élaboré un système permettant une comparaison simplifiée des niveaux d'expression de plusieurs gènes. Ce système de comparaison a nécessité la mise au point d'un coefficient, le Coefficient béta (voir table 2 ci-dessus), permettant de pondérer l'importance des différents gènes au sein du système.

Il résulte de ce qui précède que les $INDEX_{gène}$ ne peuvent prendre que les valeurs suivantes (voir tableau 3 ci-dessous) :

Tableau 3

| |
|---|
| INDEX *NME4* = (+1 ou -1) x (-1,3207) = +/- 1,3207 |
| INDEX *ITPR3* = (+1 ou -1) x (+1,0850) = +/- 1,0850 |
| INDEX *SESN3* = (+1 ou -1) x (+1,0301) = +/- 1,0301 |
| INDEX *ARL4C* = (+1 ou -1) x (+1,3577) = +/- 1,3577 |
| INDEX *RPLP1* = (+1 ou -1) x (+1,0101) = +/- 1,0101 |

Un $INDEX_{patient}$ est alors calculé, correspondant à la somme des $INDEX_{gène}$ des gènes de la combinaison de gènes marqueurs dont on a mesuré le niveau d'expression à l'étape a) des procédés selon l'invention :

$$INDEX_{patient} = \Sigma\ INDEX_{gène}$$

Cet $INDEX_{patient}$ est ensuite comparé à une valeur de seuil final de référence préalablement déterminées par la Demanderesse pour chaque combinaison de gènes marqueurs.

A titre d'exemple, pour la combinaison des 5 gènes, ladite valeur de seuil final de référence est égale à 1,102.

Ainsi, sur la base de la valeur de seuil final de référence définie pour chaque combinaison de gènes marqueurs, il est possible d'établir le pronostic de survie du patient testé.

Selon un mode de réalisation préférentiel des procédés l'invention, on mesure à l'étape a) dans un échantillon de sang préalablement prélevé d'un patient à tester, par qPCR, le niveau d'expression en nombre de cycle ($Ct_{gène}$) des 5 gènes *NME4*, *ITPR3*, *SESN3*, *ARL4C* et *RPLP1* et des deux gènes ubiquitaires B2M et GAPDH. Le niveau d'expression normalisé $\Delta Ct_{gène}$ de chacun des 5 gènes *NME4*, *ITPR3*, *SESN3*, *ARL4C* et *RPLP1* est ensuite calculé selon :

$$\Delta Ct_{gène} = Ct_{gène} - (Ct_{B2M} + Ct_{GAPDH})\ /\ 2$$

Un $INDEX_{gène}$ est ensuite calculé pour chacun des gènes selon :

- $INDEX_{gène}$ = +1*coefficient $\beta$ du gène, si $\Delta Ct_{gène}$ > seuil de référence du gène, et
- $INDEX_{gène}$ = -1*coefficient $\beta$ du gène, si $\Delta Ct_{gène}$ < seuil de référence du gène

dans lequel le coefficient $\beta$ et le seuil de référence d'un gène donné sont tels que définis dans le tableau 2 ci-après

Tableau 2

| Gène | $S_{ref}$ | Coefficient $\beta$ |
|---|---|---|
| *NME4* | 3,1864 | -1,3207 |
| *ITPR3* | 6,4014 | 1,0850 |

(suite)

| Gène | $S_{ref}$ | Coefficient $\beta$ |
|------|-----------|---------------------|
| SESN3 | 3,6262 | 1,0301 |
| ARL4C | 2,6286 | 1,3577 |
| RPLP1 | 6,3748 | 1,0101 |

L'INDEX$_{patient}$ est alors calculé comme :

$$INDEX_{patient} = INDEX_{NME4} + INDEX_{ITPR3} + INDEX_{SESN3} + INDEX_{ARL4C} + INDEX_{RPLP1}.$$

Cette valeur d'INDEX$_{patient}$ est ensuite comparée avec la valeur de seuil final de référence de la combinaison des 5 gènes marqueurs, soit 1,102.

Si un patient présente un INDEX$_{patient}$ inférieur à 1,102, il est considéré comme ayant un pronostic de survie à long terme, et peut être considéré éligible pour un traitement à base de gemcitabine. A contrario, si un patient présente un INDEX$_{patient}$ supérieur à 1,102, ceci indique qu'il présente un pronostic de survie à court terme et qu'un traitement à base de gemcitabine ne présentera pas ou peu de bénéfice. Un autre traitement devrait alors être envisagé.

L'invention concerne également une méthode de suivi de l'efficacité d'un traitement du cancer du pancréas par la mise en œuvre du procédé selon l'invention décrit précédemment, avantageusement à intervalles de temps de traitement.

L'homme du métier saura sans difficulté déterminer les intervalles de temps de traitement auxquels il souhaite mettre en œuvre le procédé selon l'invention. Par exemple, les intervalles de temps adéquats sont de 1 semaine, 2 semaines, 1 mois, 2 mois ou 6 mois.

La divulgation concerne aussi la gemcitabine pour son utilisation pour le traitement du cancer du pancréas chez un patient présentant un pronostic de survie à long terme, par exemple supérieur à 10 mois, avantageusement supérieur à 12 mois, encore plus avantageusement supérieur à 15 mois, le pronostic étant déterminé selon l'un des procédés décrits précédemment.

La divulgation concerne aussi la gemcitabine pour son utilisation pour le traitement du cancer du pancréas chez un patient présentant un INDEX$_{patient}$ inférieur à 1,102 calculé selon le mode de réalisation préférentiel décrit précédemment.

La divulgation concerne aussi la gemcitabine pour son utilisation pour le traitement du cancer du pancréas chez un patient présentant un pronostic de survie à long terme, ledit pronostic de survie à long terme étant déterminé par un procédé *in vitro* comprenant les étapes suivantes :

a) mesurer le niveau d'expression d'au moins un gène marqueur choisi parmi le groupe constitué des gènes *NME4, ITPR3, SESN3, ARL4C* et *RPLP1* ou des gènes homologues, dans un échantillon de sang dudit patient préalablement prélevé ;
b) comparer le niveau d'expression mesuré du ou des gènes marqueur(s) choisi(s) à l'étape a) chez ledit patient avec un seuil de référence;
c) évaluer le temps de survie chez ledit patient.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle on mesure à l'étape a), le niveau d'expression d'au moins deux gènes, avantageusement de 3, plus avantageusement de 4, et préférentiellement de 5 gènes.

La divulgation concerne aussi la gemcitabine pour son utilisation pour le traitement du cancer du pancréas chez un patient présentant un pronostic de survie à long terme, ledit pronostic de survie à long terme étant déterminé par l'un procédé *in vitro* tel que décrit précédemment, dans laquelle l'échantillon de sang est un échantillon de sang total périphérique.

La divulgation concerne aussi la gemcitabine pour son utilisation pour le traitement du cancer du pancréas chez un patient présentant un pronostic de survie à long terme, ledit pronostic de survie à long terme étant déterminé par l'un procédé *in vitro* tel que décrit précédemment, dans laquelle le niveau d'expression du ou des gène(s) est mesuré par la mesure de la quantité de la (ou des) protéine(s) codée(s) par ledit gène présente(s) dans l'échantillon de sang.

La divulgation concerne aussi la gemcitabine pour son utilisation pour le traitement du cancer du pancréas chez un patient présentant un pronostic de survie à long terme, ledit pronostic de survie à long terme étant déterminé par l'un procédé *in vitro* tel que décrit précédemment, dans laquelle le niveau d'expression du ou des gène(s) est mesuré par le niveau de la transcription de l'ARN ou de l'ADNc dudit gène.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle l'étape a), outre le niveau d'expression d'un gène ($Ct_{gène}$) marqueur ou de chaque gène d'une combinaison d'au moins 2 gènes marqueurs, on mesure le niveau d'expression d'au moins un gène ubiquitaire, lesdits niveaux d'expression étant mesurés en nombre de cycle(s) (Ct, «cycle threshold») par PCR quantitative en temps réel (qPCR), puis on normalise le niveau d'expression du ou des gène(s) marqueur(s) par rapport au niveau d'expression du au moins un gène ubiquitaire selon

$$\Delta Ct_{gène} = Ct_{gène} - [(\text{moyenne des}) \, Ct \, (\text{gène(s) ubiquitaire(s)})]$$

afin d'obtenir un niveau d'expression normalisé $\Delta Ct_{gène}$ pour chaque gène marqueur dont on a mesuré le niveau d'expression.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle le niveau d'expression normalisé $\Delta Ct$ d'un gène marqueur ou de chaque gène d'une combinaison d'au moins deux gènes marqueurs est normalisé par rapport à deux gènes ubiquitaires B2M et GAPDH.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle lors de l'étape a), le niveau d'expression d'un seul gène marqueur est mesuré et lors des étapes b) et c), les seuils de référence ($S_{ref}$) d'un gène donné sont :

| Gène | $S_{ref}$ |
|------|-----------|
| NME4 | 3,1864 |
| ITPR3 | 6,4014 |
| SESN3 | 3,6262 |
| ARL4C | 2,6286 |
| RPLP1 | 6,3748 |

- un niveau d'expression normalisé $\Delta Ct$ de NME4 supérieur à 3,1864 et/ou
- un niveau d'expression normalisé $\Delta Ct$ de ITPR3, SESN3, ARL4C et/ou
  RPLP1 inférieur respectivement à 6,4015 ; 3,6262 ; 2,6286 et 6,3748 indiquant un pronostic de survie à long terme.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle lors de l'étape a), on mesure le niveau d'expression d'une combinaison d'au moins deux gènes marqueurs et, lors des étapes b), pour chaque gène dont on a mesuré l'expression

i) on détermine une valeur $INDEX_{gène}$ dépendante du niveau d'expression normalisé du gène, définie par :

- $INDEX_{gène}$ = +1*coefficient $\beta$ du gène, si $\Delta Ct_{gène}$ > seuil de référence du gène, et
- $INDEX_{gène}$ = -1*coefficient $\beta$ du gène, si $\Delta Ct_{gène}$ < seuil de référence du gène, et

dans lequel le coefficient $\beta$ et le seuil de référence ($S_{ref}$) d'un gène donné sont tels que définis ci-après

| Gène | $S_{ref}$ | Coefficient $\beta$ |
|------|-----------|---------------------|
| NME4 | 3,1864 | -1,3207 |
| ITPR3 | 6,4014 | 1,0850 |

(suite)

| Gène | $S_{ref}$ | Coefficient $\beta$ |
|------|-----------|---------------------|
| *SESN3* | 3,6262 | 1,0301 |
| *ARL4C* | 2,6286 | 1,3577 |
| *RPLP1* | 6,3748 | 1,0101 |

ii) on détermine, pour un patient donné, une valeur $INDEX_{patient}$ égale à la somme des $INDEX_{gène}$ des gènes de la combinaison, et

iii) on compare l'$INDEX_{patient}$ avec un seuil final de référence préalablement déterminé pour la combinaison des gènes.

La divulgation concerne aussi la gemcitabine pour son utilisation pour le traitement du cancer du pancréas chez un patient présentant un pronostic de survie à long terme, ledit pronostic de survie à long terme étant déterminé par le procédé *in vitro* tel que décrit précédemment, dans laquelle on mesure le niveau d'expression des 5 gènes marqueurs, le seuil final de référence étant de 1,102, un $INDEX_{patient}$ inférieur à 1,102 indiquant un pronostic de survie à long terme.

La divulgation concerne aussi la gemcitabine pour son utilisation pour le traitement du cancer du pancréas chez un patient présentant un $INDEX_{patient}$ inférieur à 1,102, l'$INDEX_{patient}$ étant déterminé par le procédé selon l'invention, dans lequel on mesure le niveau d'expression des 5 gènes marqueurs, le seuil final de référence étant de 1,102, un $INDEX_{patient}$ inférieur à 1,102 indiquant un pronostic de survie à long terme.

La divulgation concerne aussi la gemcitabine pour son utilisation pour le traitement du cancer du pancréas chez un patient présentant un $INDEX_{patient}$ inférieur à 1,102, l'$INDEX_{patient}$ étant déterminé par un procédé *in vitro* comprenant les étapes suivantes :

a) mesurer le niveau d'expression d'une combinaison des cinq gènes marqueurs *NME4, ITPR3, SESN3, ARL4C* et *RPLP1* et de deux gènes ubiquitaires B2M et GADPH dans un échantillon de sang dudit patient préalablement prélevé, le niveau d'expression des gènes correspondant au niveau de transciption de l'ARN ou de l'ADNc desdits gènes, mesuré en nombre de cycle(s) (Ct, « cycle threshold ») par PCR quantitative en temps réel (qPCR), le niveau d'expression des cinq gènes marqueurs étant ensuite normalisés par rapport au niveau d'expression des gènes ubiquitaires selon

$$\Delta Ct_{gène} = Ct_{gène} - [\text{moyenne des } Ct_{gènes\ ubiquitaires}]$$

afin d'obtenir un niveau d'expression normalisé $\Delta Ct_{gène}$ pour chacun des cinq gènes marqueurs,

b) comparer le niveau d'expression mesuré des gènes marqueurs choisis à l'étape a) chez ledit patient et pour chaque gène dont on a mesuré l'expression : i)

on détermine une valeur $INDEX_{gène}$ dépendante du niveau d'expression normalisé du gène, définie par :

- $INDEX_{gène}$ = +1*coefficient $\beta$ du gène, si $\Delta Ct_{gène}$ > seuil de référence du gène, et
- $INDEX_{gène}$ = -1*coefficient $\beta$ du gène, si $\Delta Ct_{gène}$ < seuil de référence du gène, et

dans lequel le coefficient $\beta$ et le seuil de référence ($S_{ref}$) d'un gène donné sont tels que définis ci-après

| Gène | $S_{ref}$ | Coefficient $\beta$ |
|------|-----------|---------------------|
| *NME4* | 3,1864 | -1,3207 |
| *ITPR3* | 6,4014 | 1,0850 |
| *SESN3* | 3,6262 | 1,0301 |
| *ARL4C* | 2,6286 | 1,3577 |
| *RPLP1* | 6,3748 | 1,0101 |

ii) on détermine, pour un patient donné, une valeur $INDEX_{patient}$ égale à la somme des $INDEX_{gène}$ des gènes de la combinaison, et

iii)on compare l'$INDEX_{patient}$ avec le seuil final de référence de 1,102 ;

c) évaluer le temps de survie chez ledit patient, un $INDEX_{patient}$ inférieur à 1,102 indiquant un pronostic de survie à long terme.

La présente divulgation concerne en outre une puce à ADN ayant sur ses surfaces, des acides nucléiques capables de s'hybrider avec au moins un des gènes choisis dans le groupe consistant en *NME4, ITPR3, SESN3, ARL4C* et *RPLP1,* de préférence au moins 2, 3 ou 4 gènes et, plus préférentiellement, pour détecter le niveau d'expression des 5 gènes, éventuellement avec des acides nucléiques spécifiques pour au moins un gène ubiquitaire, de préférence deux gènes ubiquitaires, de manière encore plus préférée des acides nucléiques spécifiques des gènes ubiquitaires *B2M* et *GAPDH.*

La présente divulgation concerne également un kit pour la détermination du pronostic du cancer du pancréas chez un patient, comprenant des moyens pour détecter le niveau d'expression d'au moins deux gènes choisis dans le groupe consistant en *NME4, ITPR3, SESN3, ARL4C* et *RPLP1,* de préférence au moins 2, 3 ou 4 gènes et, plus préférentiellement, pour détecter le niveau d'expression des 5 gènes.

Les moyens pour détecter le niveau d'expression peuvent être par exemple une puce à ADN selon la divulgation, un jeu d'amorces et un rapporteur tel que des agents fluorescents, des sondes d'hydrolyse marquées, des balises moléculaires, des sondes d'hybridation, et des anticorps.

De préférence, le kit selon la divulgation pourra comprendre des moyens pour détecter le niveau d'expression d'une combinaison de gènes choisi dans le groupe consistant en *NME4, ITPR3, SESN3, ARL4C* et *RPLP1.*

Plus préférablement, le kit selon la divulgation pourra comprendre des moyens pour détecter toutes les combinaisons de gènes mentionnées ci-dessus, de préférence au moins 2, 3 ou 4 gènes et, plus préférentiellement, des moyens pour détecter la combinaison des 5 gènes.

Le kit peut en outre comprendre des instructions pour l'utilisation dans le procédé *in vitro* selon l'invention.

Enfin, la divulgation concerne également l'utilisation d'au moins un gène choisis dans le groupe consistant en *NME4, ITPR3, SESN3, ARL4C et RPLP1,* pour le pronostic du cancer du pancréas, en particulier, lors d'un traitement à base de gemcitabine.

De préférence, la divulgation concerne l'utilisation d'au moins l'une des combinaisons de 2 à 5 gènes sélectionnés dans le groupe consistant en *NME4, ITPR3, SESN3, ARL4C* et *RPLP1* et préférentiellement la combinaison de 3 gènes, plus préférentiellement de 4 gènes, encore plus préférentiellement des 5 gènes, pour le pronostic du cancer du pancréas, en particulier, lors d'un traitement à base de gemcitabine.

La divulgation concerne également l'utilisation d'au moins un gène choisis parmi le groupe constitué par les gènes *NME4, ITPR3, SESN3, ARL4C* et *RPLP1* ou des gènes homologues, de préférence au moins 2, 3 ou 4 gènes et, plus préférentiellement, des 5 gènes pour le pronostic d'un cancer du pancréas chez un patient, ainsi que l'utilisation d'au moins un gène choisis parmi le groupe constitué par les gènes *NME4, ITPR3, SESN3, ARL4C* et *RPLP1* ou des gènes homologues, de préférence au moins 2, 3 ou 4 gènes et, plus préférentiellement, des 5 gènes pour le suivi de l'efficacité d'un traitement du cancer du pancréas, avantageusement d'un traitement à la gemcitabine, chez un patient.

L'invention concerne l'utilisation *in vitro* d'une combinaison de gènes choisis parmi le groupe constitué par les gènes *NME4* ou un gène homologue de celui-ci et *ARL4C* ou un gène homologue de celui-ci, ou d'une combinaison de 5 gènes choisis parmi le groupe constitué des gènes *NME4* ou un gène homologue de celui-ci, *ARL4C* ou un gène homologue de celui-ci, *ITPR3* ou un gène homologue de celui-ci, *SESN3* ou un gène homologue de celui-ci et *RPLP1* ou un gène homologue de celui-ci, lesdits gènes homologues étant des gènes présentant un degré d'identité de séquence d'au moins 90% avec le gène *NME4, ARL4C, ITPR3, SESN3* ou *RPLP1* respectivement, pour le pronostic d'un cancer du pancréas, chez un patient.

L'invention concerne également l'utilisation *in vitro* d'une combinaison de gènes choisis parmi le groupe constitué par les gènes *NME4* ou un gène homologue de celui-ci et *ARL4C* ou un gène homologue de celui-ci, ou d'une combinaison de 5 gènes choisis parmi le groupe constitué des gènes *NME4* ou un gène homologue de celui-ci, *ARL4C* ou un gène homologue de celui-ci, *ITPR3* ou un gène homologue de celui-ci, *SESN3* ou un gène homologue de celui-ci et *RPLP1* ou un gène homologue de celui-ci, lesdits gènes homologues étant des gènes présentant un degré d'identité de séquence d'au moins 90% avec le gène *NME4, ARL4C, ITPR3, SESN3* ou *RPLP1* respectivement, pour le suivi de l'efficacité d'un traitement du cancer du pancréas, avantageusement d'un traitement à la gemcitabine chez un patient.

La présente invention pourra être mieux comprise et d'autres avantages et caractéristiques pourront apparaitre à la lecture des exemples suivants et des dessins annexés dans lesquels:

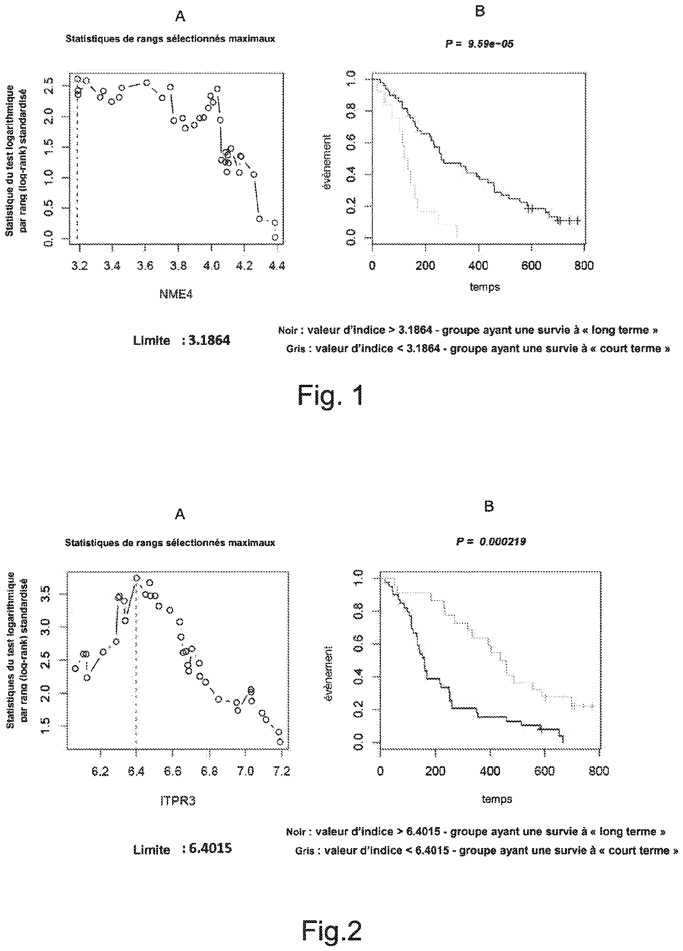

La figure 1 représente les résultats obtenus par les inventeurs dans la détermination du seuil de référence pour le gène *NME4.*

La figure 1A représente la distribution de certaines valeurs statistiques du test de Mantel-Cox en fonction de leur valeur de $\Delta$Ct. Pour déterminer la limite (cut-point) entre 2 groupes on détermine la valeur maximale obtenue par un individu pour la statistique du test de Mantel-Cox et une lecture graphique permet de déterminer la valeur du seuil de référence($S_{ref}$) de 3,1864.

La figure 1B représente les courbes de survie calculées par la méthode Kaplan-Meier pour les 2 groupes de survie à l'issue du calcul de la valeur seuil. Le groupe représenté en noir est l'ensemble des individus dont la valeur de $\Delta$Ct est inférieure à la valeur seuil ($S_{ref}$) et le groupe représenté en gris est l'ensemble des individus dont la valeur

de ∆Ct est supérieure à la valeur seuil ($S_{ref}$). Ici le groupe de longue survie est le groupe noir. La valeur-p ("pvalue") du test du log-rank est indiquée en haut du graphe : P=9,59.10⁻⁵

La figure 2 représente les résultats obtenus par les inventeurs dans la détermination du seuil de référence pour le gène *ITPR3.*

La figure 2A représente la distribution de certaines valeurs statistiques du test de Mantel-Cox en fonction de leur valeur de ∆Ct. Pour déterminer la limite (cut-point) entre 2 groupes on détermine la valeur maximale obtenue par un individu pour la statistique du test de Mantel-Cox et une lecture graphique permet de déterminer la valeur du seuil de référence ($S_{ref}$) de 6,4015.

La figure 2B représente les courbes de survie calculées par la méthode Kaplan-Meier pour les 2 groupes de survie à l'issue du calcul de la valeur seuil. Le groupe représenté en noir est l'ensemble des individus dont la valeur de ∆Ct est inférieure à la valeur seuil ($S_{ref}$) et le groupe représenté en gris est l'ensemble des individus dont la valeur de ∆Ct est supérieure à la valeur seuil ($S_{ref}$). Ici le groupe de longue survie est le groupe gris. La valeur-p du test du log-rank est indiquée en haut du graphe : P=0,000219.

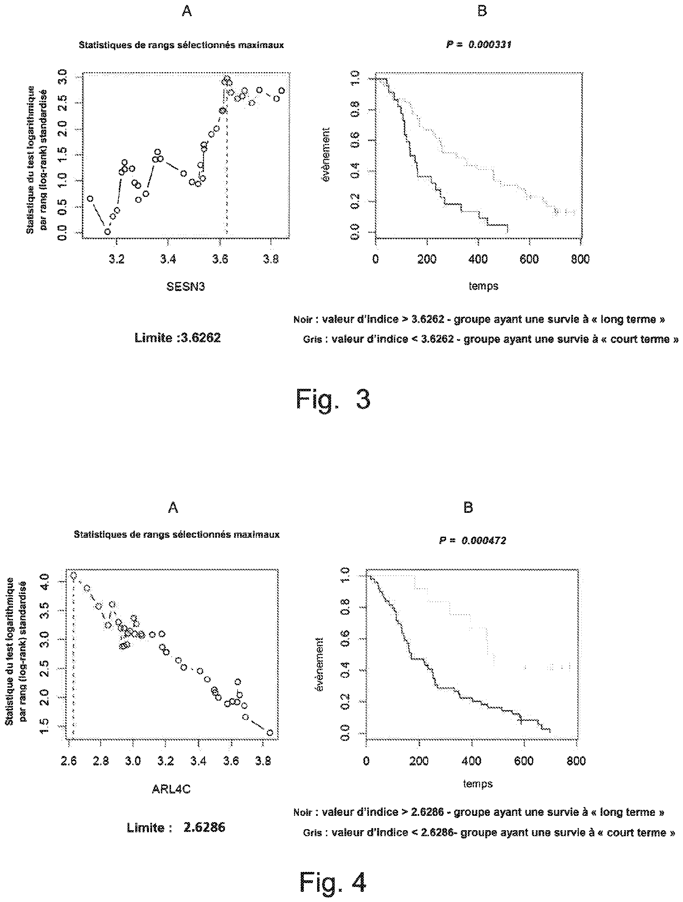

La figure 3 représente les résultats obtenus par les inventeurs dans la détermination du seuil de référence pour le gène *SESN3.*

La figure 3A représente la distribution de certaines valeurs statistiques du test de Mantel-Cox en fonction de leur valeur de ∆Ct. Pour déterminer la limite (cut-point) entre 2 groupes on détermine la valeur maximale obtenue par un individu pour la statistique du test de Mantel-Cox et une lecture graphique permet de déterminer la valeur du seuil de référence ($S_{ref}$) de 3,6262.

La figure 3B représente les courbes de survie calculées par la méthode Kaplan-Meier pour les 2 groupes de survie à l'issue du calcul de la valeur seuil. Le groupe représenté en noir est l'ensemble des individus dont la valeur de ∆Ct est inférieure à la valeur seuil ($S_{ref}$) et le groupe représenté en gris est l'ensemble des individus dont la valeur de ∆Ct est supérieure à la valeur seuil ($S_{ref}$). Ici le groupe de longue survie est le groupe gris. La valeur-p du test du log-rank est indiquée en haut du graphe : P=0,000331.

La figure 4 représente les résultats obtenus par les inventeurs dans la détermination du seuil de référence pour le gène *ARL4C.*

La figure 4A représente la distribution de certaines valeurs statistiques du test de Mantel-Cox en fonction de leur valeur de ∆Ct. Pour déterminer la limite (cut-point) entre 2 groupes on détermine la valeur maximale obtenue par un individu pour la statistique du test de Mantel-Cox et une lecture graphique permet de déterminer la valeur du seuil de référence ($S_{ref}$) de 2,6286.

La figure 4B représente les courbes de survie calculées par la méthode Kaplan-Meier pour les 2 groupes de survie à l'issue du calcul de la valeur seuil. Le groupe représenté en noir est l'ensemble des individus dont la valeur de ∆Ct est inférieure à la valeur seuil ($S_{ref}$) et le groupe représenté en gris est l'ensemble des individus dont la valeur de ∆Ct est supérieure à la valeur seuil ($S_{ref}$). Ici le groupe de longue survie est le groupe gris. La valeur-p du test du log-rank est indiquée en haut du graphe : P=0,000472.

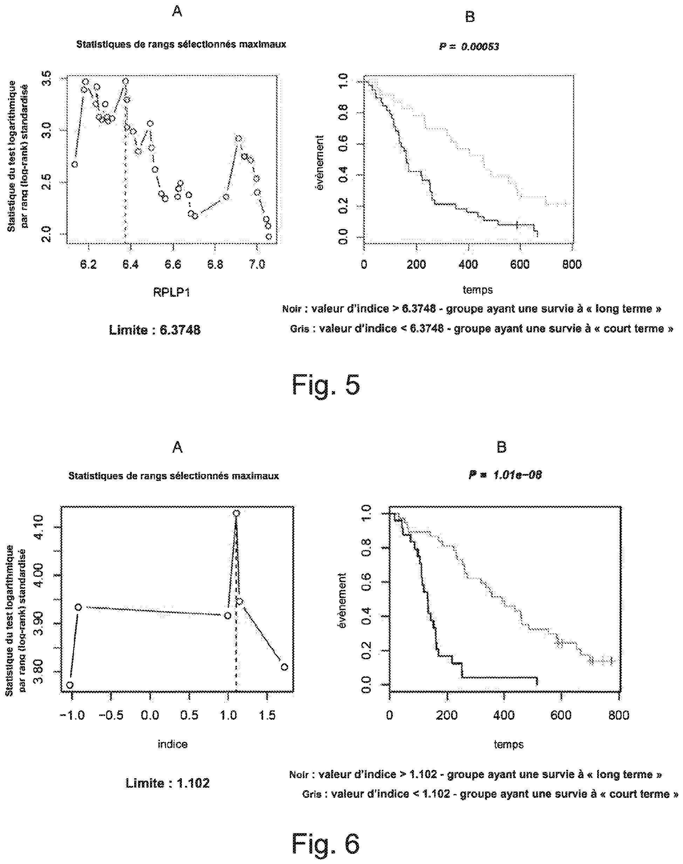

La figure 5 représente les résultats obtenus par les inventeurs dans la détermination du seuil de référence pour le gène *RPLP1.*

La figure 5A représente la distribution de certaines valeurs statistiques du test de Mantel-Cox en fonction de leur valeur de ∆Ct. Pour déterminer la limite (cut-point) entre 2 groupes on détermine la valeur maximale obtenue par un individu pour la statistique du test de Mantel-Cox et une lecture graphique permet de déterminer la valeur du seuil de référence ($S_{ref}$) de 6,3748.

La figure 5B représente les courbes de survie calculées par la méthode Kaplan-Meier pour les 2 groupes de survie à l'issue du calcul de la valeur seuil. Le groupe représenté en noir est l'ensemble des individus dont la valeur de ∆Ct est inférieure à la valeur seuil ($S_{ref}$) et le groupe représenté en gris est l'ensemble des individus dont la valeur de ∆Ct est supérieure à la valeur seuil ($S_{ref}$). Ici le groupe de longue survie est le groupe gris. La valeur-p du test du log-rank est indiquée en haut du graphe : P=0,00053.

La figure 6 représente les résultats obtenus par les inventeurs dans la détermination du seuil final de référence pour la combinaison des 5 gènes *NME4, ITPR3, SESN3, ARL4C, RPLP1.*

La figure 6A représente la distribution de certaines valeurs statistiques du test de Mantel-Cox en fonction de leur valeur de ∆Ct. Pour déterminer la limite (cut-point) entre 2 groupes, on détermine la valeur maximale obtenue par un individu pour la statistique du test de Mantel-Cox et une lecture graphique permet de déterminer la valeur du seuil de référence ($S_{ref}$) de 1,102.

La figure 6B représente les courbes de survie calculées par la méthode Kaplan-Meier pour les 2 groupes de survie à l'issue du calcul de la valeur seuil. Le groupe représenté en noir est l'ensemble des individus dont la valeur de ∆Ct est inférieure à la valeur seuil ($S_{ref}$) et le groupe représenté en gris est l'ensemble des individus dont la valeur de ∆Ct est supérieure à la valeur seuil ($S_{ref}$). Ici le groupe de longue survie est le groupe gris. La valeur-p du test du log-rank est indiquée en haut du graphe : P=1,01.10⁻⁰⁸.

EP 3 183 363 B1

## Exemple 1: Identification d'un ensemble de gènes pour le pronostic du cancer du pancréas

### 1.1 Préparation de l'ARN

Des échantillons de sang total de 61 patients prélevés avant traitement, conditionnés dans des tubes PAXgene dans de la glace sèche (expéditeur: LabConnect, Etats-Unis) ont été reçus et conservés à -80°C. Ils ont été nommés "semaine 0".

L'ARN total a été extrait à partir des 61 échantillons de sang total et nommé "semaine 0". L'analyse du transcriptome (recherche de biomarqueurs) n'a été conduite que sur ces échantillons "semaine 0".

Les 61 échantillons d'ARN ont été analysés afin de déterminer leur qualité. Le contrôle de l'intégrité de l'ARN a été réalisé avec le Bioanalyseur 2100 (Agilent Technologies, Palo Alto, États-Unis) à l'aide de la nano puce "ARN eucaryote total 6000" ("Eucaryotic Total RNA 6000 Nano Chip" d'Agilent Technologies). La quantité d'ARN a été contrôlée à l'aide d'un spectrophotomètre NanoDrop ND-1000 (THERMO Scientific). Les ARN purifiés ont été conservés à -80°C.

Un échantillon a été éliminé de l'étude en raison de la mauvaise qualité de l'ARN ("RNA Integrity Number" <8).

60 échantillons d'ARN de sang total, qui correspondent à des échantillons de sang de référence, ont été extraits à partir du sang préalablement collecté (tubes de prélèvement sanguin PAXgene, BD) en utilisant PAXgene Blood RNA Kit V.2 (PreAnalitix) selon les recommandations du fabricant.

Des expériences d'expression génique numérique (Digital Gene Expression (DGE)) ont été conduites pour sélectionner un ensemble de biomarqueurs putatifs.

La validation des biomarqueurs a été faite en utilisant une plateforme COBAS (LC480, Roche Diagnostics) et les approches biostatistiques appropriées ont été utilisées pour filtrer les meilleurs biomarqueurs.

| Sujet N° | Traitement | Morts | Survie (jours) | Survie (mois) |
|---|---|---|---|---|
| 110 | Gemcitabine | OUI | 183 | 6,0 |
| 111 | Gemcitabine | NON | 744 | 24,4 |
| 112 | Gemcitabine | OUI | 112 | 3,7 |
| 113 | Gemcitabine | NON | 589 | 19,4 |
| 207 | Gemcitabine | OUI | 98 | 3,2 |
| 208 | Gemcitabine | OUI | 87 | 2,9 |
| 211 | Gemcitabine | OUI | 160 | 5,3 |
| 508 | Gemcitabine | OUI | 253 | 8,3 |
| 805 | Gemcitabine | OUI | 654 | 21,5 |
| 1104 | Gemcitabine | OUI | 402 | 13,2 |
| 1203 | Gemcitabine | OUI | 252 | 8,3 |
| 1612 | Gemcitabine | OUI | 47 | 1,5 |
| 1613 | Gemcitabine | OUI | 73 | 2,4 |
| 2009 | Gemcitabine | OUI | 113 | 3,7 |
| 2403 | Gemcitabine | OUI | 222 | 7,3 |
| 2703 | Gemcitabine | OUI | 61 | 2,0 |
| 2704 | Gemcitabine | OUI | 134 | 4,4 |
| 3110 | Gemcitabine | OUI | 260 | 8,5 |
| 3111 | Gemcitabine | OUI | 144 | 4,7 |
| 3308 | Gemcitabine | OUI | 217 | 7,1 |
| 3309 | Gemcitabine | OUI | 112 | 3,7 |
| 3407 | Gemcitabine | OUI | 171 | 5,6 |
| 3408 | Gemcitabine | OUI | 350 | 11,5 |
| 3706 | Gemcitabine | NON | 774 | 25,4 |

(suite)

| Sujet N° | Traitement | Morts | Survie (jours) | Survie (mois) |
|---|---|---|---|---|
| 4407 | Gemcitabine | OUI | 135 | 4,4 |
| 4409 | Gemcitabine | OUI | 515 | 16,9 |
| 4410 | Gemcitabine | NON | 708 | 23,3 |
| 4411 | Gemcitabine | OUI | 105 | 3,4 |
| 4414 | Gemcitabine | OUI | 437 | 14,4 |
| 4415 | Gemcitabine | OUI | 17 | 0,6 |
| 4503 | Gemcitabine | NON | 700 | 23,0 |
| 4702 | Gemcitabine | OUI | 31 | 1,0 |
| 4902 | Gemcitabine | OUI | 161 | 5,3 |
| 4903 | Gemcitabine | NON | 602 | 19,8 |
| 5008 | Gemcitabine | OUI | 588 | 19,3 |
| 5201 | Gemcitabine | OUI | 584 | 19,2 |
| 5202 | Gemcitabine | OUI | 43 | 1,4 |
| 5331 | Gemcitabine | OUI | 699 | 23,0 |
| 5336 | Gemcitabine | OUI | 486 | 16,0 |
| 5339 | Gemcitabine | OUI | 65 | 2,1 |
| 5340 | Gemcitabine | OUI | 356 | 11,7 |
| 5341 | Gemcitabine | OUI | 120 | 3,9 |
| 5342 | Gemcitabine | OUI | 393 | 12,9 |
| 5344 | Gemcitabine | OUI | 667 | 21,9 |
| 5345 | Gemcitabine | OUI | 251 | 8,2 |
| 5346 | Gemcitabine | OUI | 163 | 5,4 |
| 5703 | Gemcitabine | OUI | 261 | 8,6 |
| 5901 | Gemcitabine | OUI | 555 | 18,2 |
| 6201 | Gemcitabine | OUI | 52 | 1,7 |
| 6303 | Gemcitabine | OUI | 269 | 8,8 |
| 8001 | Gemcitabine | OUI | 458 | 15,0 |
| 8003 | Gemcitabine | OUI | 335 | 11,0 |
| 8106 | Gemcitabine | OUI | 461 | 15,1 |
| 8901 | Gemcitabine | OUI | 460 | 15,1 |
| 9312 | Gemcitabine | OUI | 141 | 4,6 |
| 9508 | Gemcitabine | OUI | 169 | 5,6 |
| 9509 | Gemcitabine | OUI | 318 | 10,4 |
| 9901 | Gemcitabine | OUI | 153 | 5,0 |
| 10303 | Gemcitabine | OUI | 131 | 4,3 |
| 10304 | Gemcitabine | OUI | 234 | 7,7 |
| 11207 | Gemcitabine | OUI | 231 | 7,6 |

**1.2. Construction de la bibliothèque DGE et cartographie balise-gène**

Les échantillons d'ARN ont été divisés en 2 groupes :

- M4 : correspondant aux patients ayant reçu, après prélèvement des échantillons de sang de référence, de la Gemcitabine et morts avant le mois 4 ;
- M15 : correspondant aux patients ayant reçu, après prélèvement des échantillons de sang de référence, de la Gemcitabine et vivants après le mois 15.

Pour chaque groupe, des quantités équimolaires de chaque échantillon d'ARN total des patients ont été mélangées afin de constituer un pool d'ARN total.

6 bibliothèques de type DGE (technique répétée trois fois pour chaque groupe) ont été construites à partir desdits pool.

Les bibliothèques ont été construites avec le kit prévu à cet effet de la société Illumina (DGE Tag profiling kit) selon le protocole du fabricant (version 2.1B), en utilisant 2 $\mu$g d'ARN total.

Le séquençage et les analyses subséquentes ont été effectués en utilisant le dispositif Pipeline d'Illumina.

La plate-forme MGX (Montpellier, France) a été utilisée.

Les données de chaque bibliothèque DGE ont été analysés avec le logiciel de BIOTAG (Acobiom, Montpellier, France) pour la détection et le comptage des balises, et l'évaluation de la qualité des bibliothèques DGE (Piquemal et al., 2002).

**1.3. Marquage et sélection de balises**

Une base de données compilant des séquences d'*Homo sapiens* et des informations connexes a été générée à partir de séquences issues de la base de données UniGene (Built#232, Mars 2012, NCBI).

Pour chaque séquence de la base de données, la balise DGE attendue (balise canonique) située en amont du site de restriction NlaIII (CATG) le plus proche de l'extrémité 3' de la séquence (R1), ainsi que les balises putatives situées à des positions intérieures (étiqueté R2, R3 et R4 à partir de l'extrémité 3' du transcrit), ont été extraits (Piquemal et al., 2002).

Des balises expérimentales obtenues à partir des bibliothèques DGE ont été appariées et marquées (correspondances exactes pour le 17 pb) à l'aide de cette collection de balises virtuelles. Une correspondance pour chaque balise expérimentale avec les balises canoniques virtuelles (R1) a été recherchée en premier lieu. Ensuite, les balises expérimentales non-appariées avec les balises R2, puis avec R3 et R4 ont été marquées.

Les analyses des expériences DGE ont été réalisées en utilisant la méthode edgeR (version 2.6.9, Bioconductor). Les gènes analysés ont été choisis en fonction de (1) filtres mathématiques ayant le plus grand facteur de variation différentiel («differential Fold Change», >1,5),

Le taux de faux positifs (FDR) est une valeur-p ("pvalue") ajustée (ici FDR < 10% est posé) qui se calcule d'après l'erreur de type 1 ($\alpha$=5%) rapportée dans les considérations générales. (J.R. Statist. Soc. B, 2010, Discovering the false discovery rate, Y Benjamini)

(2) filtres biologiques avec la mise en œuvre de gènes ciblés dans des processus spécifiques et des voies métaboliques connues.

**1.4. Synthèse de l'ADNc pour la qPCR**

Des transcriptions inverses ont été réalisées pour chacun des 60 échantillons d'ARNs dans 20 $\mu$l de volume de réaction final avec 300 ng d'ARN total en utilisant 200 unités d'enzyme SuperScript II (M-MLV type TA, Invitrogen) et 250 ng d'amorces aléatoires selon les instructions du fabricant (25°C, 10 min, 42°C, 50 min, 70°C 15 min), le même jour avec la même pipette et les mêmes manipulateur.

**1.5. qPCR**

La validation des gènes ciblés a été réalisée par qPCR sur une plate-forme de Roche Diagnostics.

Les expériences de qPCR ont été réalisées en utilisant un mélange LightCycler® 480 ADN SYBR Green Master mix (Roche Diagnostics) sur un appareil Roche Diagnostics LightCycler480® selon les instructions du fabricant.

Pour les dosages au SYBR Green, le mélange réactionnel a été préparé comme suit dans un volume final de 10 $\mu$l : 5 $\mu$l de LightCycler 480 DNA SYBR Green Master 2X (Roche), 4 $\mu$l de couples d'amorces à 50 $\mu$M (Eurogentec), 1 $\mu$l de matrice d'ADNc (1/15 dilution finale).

Le programme de la PCR consiste en une première étape de pré-incubation à 95°C pendant 10 min suivie de 45 cycles de PCR (95°C pendant 10 s, 63°C pendant 15 s et 72°C pendant 15 s).

Pour discriminer les produits spécifiques des produits non spécifiques et des dimères d'amorces, une fusion a été obtenue par augmentation progressive de la température 65 à 97°C.

Tableau 4 : amorces correspondant aux 5 biomarqueurs utilisés pour la qPCR ainsi que les 2 gènes de référence (*gènes ubiquitaires).

| Biomarqueur | Amorce sens | Amorce anti-sens |
|---|---|---|
| NME4 | CATGATTGGACACACCGACTC (SEQ ID NO. 1) | GACGCTGAAGTCACCCCTTAT (SEQ ID NO. 2) |
| ITPR3 | TCCTGGGGAAGAGTTGTACG (SEQ ID NO. 3) | AGGAGAAAAACAAGCGGTCA (SEQ ID NO. 4) |
| SESN3 | TTGCCTTTGTAGTCCTGTGC (SEQ ID NO. 5) | CATTAGTCCAGTCACGTGCTTC (SEQ ID NO. 6) |
| ARL4C | AAAGCCCTGTGGTGTATCAA (SEQ ID NO. 7) | GCTTCCTCTGTTGGGTCAGA (SEQ ID NO. 8) |
| RPLP1 | TGGGCTTTGGTCTTTTTGAC (SEQ ID NO. 9) | CAGACCATTTTTGCAGAGCA (SEQ ID NO. 10) |
| B2M* | GCTCAGTAAAGACACAACCATCC (SEQ ID NO. 11) | CATCTGTGGATTCAGCAAACC (SEQ ID NO. 12) |
| GAPDH* | ATGGGGAAGGTGAAGGTCG (SEQ ID NO. 13) | GGGGTCATTGATGGCAACAATA (SEQ ID NO. 14) |

Les données de la qPCR ont été analysées en utilisant la méthode Delta.Ct (ΔCt) (Livak et Schmittgen, 2001). Pour tous les gènes cibles, les valeurs ΔCt ont été déterminées par application de la formule

$$\Delta Ct = Ct\ (\text{gène}) - [Ct\ (B2M) + Ct\ (GAPDH)]\ /\ 2$$

Les deux gènes ubiquitaires sont :

- *B2M* (NM_009735, bêta-2 microglobuline de *Mus musculus,* ARNm) et
- *GAPDH* (NM_002046, glycéraldéhyde-3-phosphate déshydrogénase, variant de transcription 1, ARNm + NM_001256799 glycéraldéhyde-3-phosphate déshydrogénase d'*Homo sapiens*, variant de transcription 2, ARNm).

### 1.6. Résultats

Identification de l'empreinte génétique

Par utilisation de la méthode DGE, les profils transcriptomiques de sang total de patients a été réalisée et 169 gènes ont été sélectionnés avec la méthode edgeR telle que décrit au point 1.3.

Dans un essai de qPCR, une réaction positive est détectée par une accumulation d'un signal fluorescent.

Le Ct étant défini comme le nombre de cycles de PCR nécessaires pour que le signal fluorescent franchisse le bruit de fond, la valeur de Ct est inversement proportionnelle à la quantité d'acide nucléique cible dans l'échantillon. Ainsi plus faible est la valeur de Ct, plus élevé est la quantité d'acide nucléique cible dans l'échantillon.

Une étude pharmacogénomique accessoire a par ailleurs été réalisée. Des échantillons de l'ARN de sang de 61 patients ont été prélevés avant tout traitement, et ils ont été analysés par RT-PCR.

Une empreinte génétique pro-métastatique («genetic pro-metastatic fingerprint») présente dans 65% des patients, hautement prédictive de la survie globale, et, en outre, en interaction avec le type de traitement, a été mise en évidence. Les patients traités à la Gemcitabine, présentant l'empreinte génétique pro-métastatique ont un faible taux de survie globale (OS) (5,0 mois).

Les gènes *NME4, ITPR3, SESN3, ARL4C* et *RPLP1* ont été sélectionnés par les inventeurs parmi les 169 gènes, en accord avec la nature multifactorielle de cette indication.

| Nom | Description | Identifiant Ensembl | Identifiant Genbank (exemple d'ARNm) |
|---|---|---|---|
| *NME4* | Homo sapiens NME/NM23 nucléoside diphosphate kinase 4 | ENSG00000103202 | ENSDG00000103202 (SEQ ID NO. 15) NM_001286433.1 (SEQ ID NO. 16) NM_001286439.1 (SEQ ID NO. 17) NM_001286440.1 (SEQ ID NO. 18) NM_001286438.1 (SEQ ID NO. 19) NM_001286436.1 (SEQ ID NO. 20) NM_005009.2 (SEQ ID NO. 21) NM_001286435.1 (SEQ ID NO.22) |
| *ITPR3* | inositol 1,4,5-triphosphate récepteur, de type 3 | ENSG00000096433 | ENSG00000096433 (SEQ ID NO. 23) NM_002224.3 (SEQ ID NO. 24) XM_011514576.1 (SEQ ID NO. 25) XM_011514577.1 (SEQ ID NO. 26) |
| *SESN3* | sestrine 3 | ENSG00000149212 | ENSG00000149212 (SEQ ID NO. 27) NM_001271594.1 (SEQ ID NO. 28) NM_144665.3 (SEQ ID NO. 29) |
| *ARL4C* | ADP-ribosylation factor like 4C | ENSG00000188042 | NM_005737 (SEQ ID NO. 30) AJ579850* (SEQ ID NO. 31) AJ57985* (SEQ ID NO. 32) NM_001282431.1 (SEQ ID NO. 33) NM_005737.3 (SEQ ID NO. 34) |
| *RPLP1* | Large protéine ribosomique P1 | ENSG00000137818 | NM_001003 (SEQ ID NO. 35) NM_213725 (SEQ ID NO. 36) NM_001003.2 (SEQ ID NO. 37) NM_213725.1 (SEQ ID NO. 38) |

(suite)

| Nom | Description | Identifiant Ensembl | Identifiant Genbank (exemple d'ARNm) |
|---|---|---|---|
| *GAPDH* | glycéraldéhyde-3-phosphate déshydrogénase | ENSG00000111640 | NM_002046 (SEQ ID NO. 39) NM_001256799 (SEQ ID NO. 40) |
| *B2M* | bêta-2 microglobuline | ENSG00000166710 | NM_009735 (SEQ ID NO. 41) |
| *: Variants d'épissage du transcrit du gène ARL7 (Homo sapiens mRNA for ADP ribosylation factor-like protein 7 (ARL7 gene), splice variant 1 (ENSG00000188042), connus comme homonymes d'ARL4C. | | | |

L'identification de l'empreinte génétique décrite ici ouvre une nouvelle voie à la thérapie personnalisée dans cette indication.

L'empreinte génétique pro-métastatique, basée sur une valeur spécifique de Delta.Ct ($\Delta$Ct) peut être habituellement déterminée par RT-PCT à partir d'ARN d'échantillons de sang. La valeur $\Delta$Ct illustrant le niveau d'expression d'un gène donné chez un patient donné est obtenue par amplification par RT-PCR du gène donné et après normalisation par rapport aux gènes de référence (B2M, GAPDH). Plus faible est la valeur de $\Delta$Ct, plus élevé est le niveau d'expression du gène.

Les patients ayant un pronostic de survie élevé et/ou éligible à un traitement par la Gemcitabine présente une valeur INDEX spécifique.

Détermination des INDEX

Plus précisément, chaque gène identifié (*NME4, ITPR3, SESN3, ARL4C* et *RPLP1*), a été évalué pour sa valeur pronostique pour la survie globale sur une population type de patients atteints d'un cancer du pancréas (population de référence), avant que lesdits patients ne reçoivent un traitement (voir point 1.1).

Sur ladite population référence et pour les 5 gènes identifiés (*NME4, ITPR3, SESN3, ARL4C* et *RPLP1*), une valeur seuil de $\Delta$CT a été définie par qPCR.

Ensuite, pour les 5 gènes identifiés (*NME4, ITPR3, SESN3, ARL4C* et *RPLP1*), un modèle de Mantel-Cox (ou test de Log-Rank, « test logarithmique par rang ») a testé la différence de survie entre la survie à long terme et la survie à court terme, et a établi un "seuil de référence" ($S_{ref}$) et un "coefficient bêta" pour chaque gène identifié. Ce test du Log-rank permet de comparer deux groupes dont les courbes de survie ont été calculées par la méthode de Kaplan Meier. On teste l'hypothèse nulle H0 d'égalité des fonctions de survie dans les deux groupes considérés. Sous H0, le risque de décès étant le même à un temps donné Ti entre les deux groupes, on s'attend donc à une proportion de décès identique dans ceux-ci (Bland & Altman, 2004).

Le tableau 2 ci-dessous présente les valeurs du seuil de référence ($S_{ref}$) et du coefficient bêta obtenues pour les 5 gènes *NME4, ITPR3, SESN3, ARL4C* et *RPLP1,* sur la population référence.

Tableau 2 : seuil et coefficient bêta (coefficient $\beta$) pour les 5 gènes identifiés

| Nom | $S_{ref}$ | Coefficient $\beta$ |
|---|---|---|
| *NME4* | 3,1864 | -1,3207 |
| *ITPR3* | 6,4014 | 1,0850 |
| *SESN3* | 3,6262 | 1,0301 |
| *ARL4C* | 2,6286 | 1,3577 |
| *RPLP1* | 6,3748 | 1,0101 |

Un niveau d'expression normalisé $\Delta$Ct de NME4 supérieur à 3,1864, et/ou

- un niveau d'expression normalisé $\Delta$Ct de ITPR3, SESN3, ARM4C et/ou RPLP1 inférieur respectivement à 6,4015; 3,6262; 2,6286 et 6,3748, indique un pronostic de survie à long terme, tandis que
- un niveau d'expression normalisé $\Delta$Ct de NME4 inférieur à 3,1864, et/ou

- un niveau d'expression normalisé $\Delta$Ct de ITPR3, SESN3, ARM4C et/ou RPLP1 supérieur respectivement à 6,4015 ; 3,6262 ; 2,6286 et 6,3748, indique un pronostic de survie à court terme.

Le pronostic peut être affiné en prenant une combinaison des 5 gènes du tableau 2.

La valeur $\Delta$CT obtenue est alors corrigée en une variable qui peut prendre deux valeurs :

- +1 si la valeur $\Delta$CT des échantillons est strictement supérieur à la valeur de $S_{ref}$ définie pour le gène considéré ;
- -1 si la valeur $\Delta$CT des échantillons est strictement inférieur à la valeur de $S_{ref}$ définie pour le gène considéré.

Cela permet alors de définir pour chaque gène considéré une valeur INDEX qui se calcul selon : INDEX = (+1 ou -1) x (coefficient $\beta$).

Ainsi, pour les gènes considérés, l'INDEX calculé sur la population de référence de patients atteint d'un cancer du pancréas peut prendre les valeurs données dans le tableau 3 suivant :

Tableau 3

| |
|---|
| INDEX *NME4* = (+1 ou -1) x (-1,3207) = +/- 1,3207 |
| INDEX *ITPR3* = (+1 ou -1) x (+1,0850) = +/- 1,0850 |
| INDEX *SESN3* = (+1 ou -1) x (+1,0301) = +/- 1,0301 |
| INDEX *ARL4C* = (+1 ou -1) x (+1,3577) = +/- 1,3577 |
| INDEX *RPLP1* = (+1 ou -1) x (+1,0101) = +/- 1,0101 |

C'est l'INDEX de la combinaison de gènes retenu pour l'évaluation du pronostic de survie du patient qui va alors permettre d'établir le pronostic.

Le modèle de Mantel-Cox (ou test de Log-Rank) prenant en compte les INDEX calculés a permis de déterminer un "seuil final de référence" pour chacune des combinaisons, qui va permettre d'établir le pronostic de survie du patient testé.

La valeur du seuil final de référence pour la combinaison des 5 gènes considérés est égale à 1,102.

Sur la base de la valeur de seuil final de référence définie pour chaque gène ou pour chaque combinaison, il est possible d'établir le pronostic de survie du patient test :

- si l'INDEX$_{patient}$ calculé chez le patient testé est inférieur à la valeur du seuil final de référence alors le pronostic est une survie à long terme et
- si l'INDEX$_{patient}$ calculé chez le patient testé est supérieur à la valeur du seuil final de référence alors le pronostic est une survie à court terme.

## Revendications

1. Procédé *in vitro* pour établir un pronostic de survie chez un patient atteint d'un cancer du pancréas comprenant les étapes suivantes :

   a) mesurer le niveau d'expression de chaque gène d'une combinaison de gènes marqueurs choisis parmi le groupe constitué des gènes *NME4* ou un gène homologue de celui-ci et *ARL4C* ou un gène homologue de celui-ci, lesdits gènes homologues étant des gènes présentant un degré d'identité de séquence d'au moins 90% avec le gène *NME4* ou *ARL4C* respectivement, dans un échantillon de sang dudit patient préalablement prélevé ;
   b) comparer le niveau d'expression mesuré des gènes marqueurs choisis à l'étape a) chez ledit patient avec un seuil de référence;
   c) évaluer le temps de survie chez ledit patient.

2. Procédé *in vitro* pour prévoir ou évaluer l'efficacité et/ou le bénéfice d'un traitement du cancer du pancréas chez un patient atteint de ladite pathologie comprenant les étapes suivantes :

   a) mesurer le niveau d'expression de chaque gène d'une combinaison de gènes marqueurs choisis parmi le groupe constitué des gènes *NME4* ou un gène homologue de celui-ci et *ARL4C* ou un gène homologue de celui-ci, lesdits gènes homologues étant des gènes présentant un degré d'identité de séquence d'au moins 90% avec le gène *NME4* ou *ARL4C* respectivement, dans un échantillon de sang dudit patient préalablement prélevé ;

b) comparer le niveau d'expression mesuré des gènes marqueurs choisis à l'étape a) chez ledit patient avec un seuil de référence ;

c) évaluer le temps de survie chez ledit patient.

**3.** Procédé selon la revendication 2, **caractérisé en ce que** ledit traitement est un traitement à la gemcitabine.

**4.** Procédé selon l'une quelconque des revendications 1 à 3, **caractérisé en ce qu'**on mesure à l'étape a) le niveau d'expression de chaque gène d'une combinaison de gènes marqueurs choisis parmi le groupe constitué des gènes *NME4* ou un gène homologue de celui-ci, *ARL4C* ou un gène homologue de celui-ci, *ITPR3* ou un gène homologue de celui-ci, SESN3 ou un gène homologue de celui-ci et *RPLP1* ou un gène homologue de celui-ci, lesdits gènes homologues étant des gènes présentant un degré d'identité de séquence d'au moins 90% avec le gène *NME4, ARL4C, ITPR3, SESN3* ou *RPLP1* respectivement.

**5.** Procédé selon l'une quelconque des revendications 1 à 4, dans lequel ledit échantillon de sang est un échantillon de sang total périphérique.

**6.** Procédé selon l'une quelconque des revendications 1 à 5, dans lequel le niveau d'expression des gènes est mesuré par la mesure de la quantité des protéines codées par lesdits gènes présentes dans l'échantillon de sang.

**7.** Procédé selon l'une quelconque des revendications 1 à 5, dans lequel le niveau d'expression des gènes est mesuré par le niveau de la transcription de l'ARN ou de l'ADNc desdits gènes.

**8.** Méthode de suivi de l'efficacité d'un traitement du cancer du pancréas, avantageusement d'un traitement à la gemcitabine, par la mise en œuvre du procédé décrit dans l'une quelconque des revendications 2 à 7, avantageusement à intervalles de temps de traitement.

**9.** Utilisation *in vitro* d'une combinaison de gènes choisis parmi le groupe constitué par les gènes *NME4* ou un gène homologue de celui-ci et *ARL4C* ou un gène homologue de celui-ci, ou d'une combinaison de 5 gènes choisis parmi le groupe constitué des gènes *NME4* ou un gène homologue de celui-ci, *ARL4C* ou un gène homologue de celui-ci, *ITPR3* ou un gène homologue de celui-ci, *SESN3* ou un gène homologue de celui-ci et *RPLP1* ou un gène homologue de celui-ci, lesdits gènes homologues étant des gènes présentant un degré d'identité de séquence d'au moins 90% avec le gène *NME4, ARL4C, ITPR3, SESN3* ou *RPLP1* respectivement, pour le pronostic d'un cancer du pancréas, chez un patient.

**10.** Utilisation *in vitro* d'une combinaison de gènes choisis parmi le groupe constitué par les gènes *NME4* ou un gène homologue de celui-ci et *ARL4C* ou un gène homologue de celui-ci, ou d'une combinaison de 5 gènes choisis parmi le groupe constitué des gènes *NME4* ou un gène homologue de celui-ci, *ARL4C* ou un gène homologue de celui-ci, *ITPR3* ou un gène homologue de celui-ci, *SESN3* ou un gène homologue de celui-ci et *RPLP1* ou un gène homologue de celui-ci, lesdits gènes homologues étant des gènes présentant un degré d'identité de séquence d'au moins 90% avec le gène *NME4, ARL4C, ITPR3, SESN3* ou *RPLP1* respectivement, pour le suivi de l'efficacité d'un traitement du cancer du pancréas, avantageusement d'un traitement à la gemcitabine chez un patient.

**Patentansprüche**

**1.** *In-vitro*-Verfahren zum Erstellen einer Überlebensprognose bei einem von Bauchspeicheldrüsenkrebs betroffenen Patienten, welches die folgenden Schritte umfasst:

a) Messen des Expressionsniveaus jedes Gens einer Kombination von Marker-Genen, welche aus der Gruppe ausgewählt sind, die aus den Genen *NME4* oder einem dazu homologen Gen und *ARL4C* oder einem dazu homologen Gen besteht, wobei die homologen Gene einen Grad von mindestens 90% Sequenzidentität mit dem Gen *NME4* beziehungsweise *ARL4C* aufweisende Gene sind, in einer zuvor entnommenen Blutprobe des Patienten;

b) Vergleichen des in Schritt a) gemessenen Expressionsniveaus der ausgewählten Marker-Gene bei dem Patienten mit einem Referenz-Schwellenwert;

c) Bewerten der Überlebenszeit bei dem Patienten.

**2.** *In-vitro*-Verfahren zur Voraussage oder Bewertung der Wirksamkeit und/oder des Nutzens einer Behandlung von Bauchspeicheldrüsenkrebs bei einem von dieser Erkrankung betroffenen Patienten, welches die folgenden Schritte umfasst:

a) Messen des Expressionsniveaus jedes Gens einer Kombination von Marker-Genen, welche aus der Gruppe ausgewählt sind, die aus den Genen *NME4* oder einem dazu homologen Gen und *ARL4C* oder einem dazu homologen Gen besteht, wobei die homologen Gene einen Grad von mindestens 90% Sequenzidentität mit dem Gen *NME4* beziehungsweise *ARL4C* aufweisende Gene sind, in einer zuvor entnommenen Blutprobe des Patienten;
b) Vergleichen des in Schritt a) gemessenen Expressionsniveaus der ausgewählten Marker-Gene bei dem Patienten mit einem Referenz-Schwellenwert;
c) Bewerten der Überlebenszeit bei dem Patienten.

**3.** Verfahren nach Anspruch 2, **dadurch gekennzeichnet, dass** die Behandlung eine Behandlung mit Gemcitabin ist.

**4.** Verfahren nach einem der Ansprüche 1 bis 3, **dadurch gekennzeichnet, dass** man in Schritt a) das Expressionsniveau jedes Gens einer Kombination von Marker-Genen misst, welche aus der Gruppe ausgewählt sind, die aus den Genen *NME4* oder einem dazu homologen Gen, *ARL4C* oder einem dazu homologen Gen, *ITPR3* oder einem dazu homologen Gen, *SESN3* oder einem dazu homologen Gen und *RPLP1* oder einem dazu homologen Gen besteht, wobei die homologen Gene einen Grad von mindestens 90% Sequenzidentität mit dem Gen *NME4, ARL4C, ITPR3,* SESN3 beziehungsweise *RPLP1* aufweisende Gene sind.

**5.** Verfahren nach einem der Ansprüche 1 bis 4, in welchem die Blutprobe eine Probe von peripherem Vollblut ist.

**6.** Verfahren nach einem der Ansprüche 1 bis 5, in welchem das Expressionsniveau der Gene durch Messung der Menge der durch die Gene kodierten Proteine gemessen wird, die in der Blutprobe vorhanden sind.

**7.** Verfahren nach einem der Ansprüche 1 bis 5, in welchem das Expressionsniveau der Gene über das Transkriptionsniveau der RNA oder der cDNA der Gene gemessen wird.

**8.** Verfahren zur Überwachung der Wirksamkeit einer Behandlung von Bauchspeicheldrüsenkrebs, vorteilhafterweise einer Behandlung mit Gemcitabin, durch Anwendung des in einem der Ansprüche 2 bis 7 beschriebenen Verfahrens, vorteilhafterweise in Intervallen der Behandlungszeit.

**9.** *In-vitro*-Verwendung einer Kombination von Genen, welche aus der Gruppe ausgewählt sind, die aus den Genen *NME4* oder einem dazu homologen Gen und *ARL4C* oder einem dazu homologen Gen besteht, oder einer Kombination von 5 Genen, welche aus der Gruppe ausgewählt sind, die aus den Genen *NME4* oder einem dazu homologen Gen, *ARL4C* oder einem dazu homologen Gen, *ITPR3* oder einem dazu homologen Gen, *SESN3* oder einem dazu homologen Gen und *RPLP1* oder einem dazu homologen Gen besteht, wobei die homologen Gene einen Grad von mindestens 90% Sequenzidentität mit dem Gen *NME4, ARL4C, ITPR3,* SESN3 beziehungsweise *RPLP1* aufweisende Gene sind, zur Prognose von Bauchspeicheldrüsenkrebs bei einem Patienten.

**10.** *In-vitro*-Verwendung einer Kombination von Genen, welche aus der Gruppe ausgewählt sind, die aus den Genen *NME4* oder einem dazu homologen Gen und *ARL4C* oder einem dazu homologen Gen besteht, oder einer Kombination von 5 Genen, welche aus der Gruppe ausgewählt sind, die aus den Genen *NME4* oder einem dazu homologen Gen, *ARL4C* oder einem dazu homologen Gen, *ITPR3* oder einem dazu homologen Gen, *SESN3* oder einem dazu homologen Gen und *RPLP1* oder einem dazu homologen Gen besteht, wobei die homologen Gene einen Grad von mindestens 90% Sequenzidentität mit dem Gen *NME4, ARL4C, ITPR3,* SESN3 beziehungsweise *RPLP1* aufweisende Gene sind, zur Überwachung der Wirksamkeit einer Behandlung von Bauchspeicheldrüsenkrebs, vorteilhafterweise einer Behandlung mit Gemcitabin, bei einem Patienten.

**Claims**

**1.** *In-vitro* method for establishing a survival prognosis for a patient suffering from pancreatic cancer, comprising the following steps:

a) measuring the level of expression of each gene of a combination of marker genes selected from the group constituted by the *NME4* genes or a homologous gene and *ARL4C* or a homologous gene, said homologous genes being genes having a degree of sequence identity of at least 90% with the *NME4* or *ARL4C* gene respectively, in a blood sample taken previously from said patient;

b) comparing the level of expression measured for the marker genes selected in step a) in said patient against a reference threshold;

c) evaluating the survival time for said patient.

2. *In-vitro* method for predicting or evaluating the effectiveness and/or benefit of pancreatic cancer treatment in a patient suffering from said pathology, comprising the following steps:

a) measuring the level of expression of each gene of a combination of marker genes selected from the group constituted by the *NME4* genes or a homologous gene and *ARL4C* or a homologous gene, said homologous genes being genes having a degree of sequence identity of at least 90% with the *NME4* or *ARL4C* gene respectively, in a blood sample taken previously from said patient;

b) comparing the level of expression measured for the marker genes selected in step a) in said patient against a reference threshold;

c) evaluating the survival time for said patient.

3. Method according to claim 2, **characterized in that** said treatment is treatment with gemcitabine.

4. Method according to any one of claims 1 to 3, **characterized in that** at step a) the level of expression is measured of each gene of a combination of marker genes selected from the group constituted by the genes *NME4* or a homologous gene, *ARL4C* or a homologous gene, *ITPR3* or a homologous gene, *SESN3* or a homologous gene and *RPLP1* or a homologous gene, said homologous genes being genes having a degree of sequence identity of at least 90% with the *NME4, ARL4C, ITPR3, SESN3* or *RPLP1* gene respectively.

5. Method according to any one of claims 1 to 4, in which said blood sample is a peripheral whole blood sample.

6. Method according to any one of claims 1 to 5, in which the level of expression of the genes is measured by measuring the quantity of proteins encoded by said genes present in the blood sample.

7. Method according to any one of claims 1 to 5, in which the level of expression of the genes is measured by the transcription level of the RNA or cDNA of said genes.

8. Method for monitoring the effectiveness of a treatment for pancreatic cancer, advantageously a treatment with gemcitabine, by implementing the method described in any one of claims 2 to 7, advantageously at treatment time intervals.

9. *In vitro* use of a combination of genes selected from the group constituted by the genes *NME4* or a homologous gene and *ARL4C* or a homologous gene, or of a combination of 5 genes selected from the group constituted by the genes *NME4* or a homologous gene, *ARL4C* or a homologous gene, *ITPR3* or a homologous gene, *SESN3* or a homologous gene and *RPLP1* or a homologous gene, said homologous genes being genes having a degree of sequence identity of at least 90% with the NME4, ARL4C, ITPR3, SESN3 or RPLP1 gene respectively, for the prognosis of pancreatic cancer, in a patient.

10. *In vitro* use of a combination of genes selected from the group constituted by the genes *NME4* or a homologous gene and *ARL4C* or a homologous gene, or of a combination of 5 genes selected from the group constituted by the genes *NME4* or a homologous gene, *ARL4C* or a homologous gene, *ITPR3* or a homologous gene, *SESN3* or a homologous gene and *RPLP1* or a homologous gene, said homologous genes being genes having a degree of sequence identity of at least 90% with the NME4, ARL4C, ITPR3, SESN3 or RPLP1 gene respectively, for monitoring the effectiveness of a treatment for pancreatic cancer, advantageously a treatment with gemcitabine in a patient.

A

Statistiques de rangs sélectionnés maximaux

B

P = 9.59e-05

NME4

temps

Limite : 3.1864

Noir : valeur d'indice > 3.1864 - groupe ayant une survie à « long terme »
Gris : valeur d'indice < 3.1864 - groupe ayant une survie à « court terme »

# Fig. 1

A

Statistiques de rangs sélectionnés maximaux

B

P = 0.000219

ITPR3

temps

Limite : 6.4015

Noir : valeur d'indice > 6.4015 - groupe ayant une survie à « long terme »
Gris : valeur d'indice < 6.4015 - groupe ayant une survie à « court terme »

# Fig.2

A

B

Statistiques de rangs sélectionnés maximaux

*P = 0.000331*

SESN3

Limite :3.6262

Noir : valeur d'indice > 3.6262 - groupe ayant une survie à « long terme »

Gris : valeur d'indice < 3.6262 - groupe ayant une survie à « court terme »

Fig. 3

A

B

Statistiques de rangs sélectionnés maximaux

*P = 0.000472*

ARL4C

Limite : 2.6286

Noir : valeur d'indice > 2.6286 - groupe ayant une survie à « long terme »

Gris : valeur d'indice < 2.6286- groupe ayant une survie à « court terme »

Fig. 4

24

A

Statistiques de rangs sélectionnés maximaux

B

P = 0.00053

RPLP1

Limite : 6.3748

temps

Noir : valeur d'indice > 6.3748 - groupe ayant une survie à « long terme »
Gris : valeur d'indice < 6.3748 - groupe ayant une survie à « court terme »

Fig. 5

A

Statistiques de rangs sélectionnés maximaux

B

P = 1.01e~08

indice

temps

Limite : 1.102

Noir : valeur d'indice > 1.102 - groupe ayant une survie à « long terme »
Gris : valeur d'indice < 1.102 - groupe ayant une survie à « court terme »

Fig. 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **A. TSIAOUSIDOU et al.** *Pancreatology,* 2013, vol. 13, 564-569
- **H. FUJITA et al.** *Neoplasia,* 2010, vol. 12, 807-817
- **T. TAKADATE et al.** *Clinical Proteomics,* 2012, vol. 9, 8
- **A. KRACMAROVA et al.** *Leukemia & Lymphoma,* 2008, vol. 49 (7), 1297-1305
- **Y BENJAMINI.** Discovering the false discovery rate. *J.R. Statist. Soc. B,* 2010